# EUROPEAN PATENT APPLICATION

(11) **EP 1 547 594 A1**
(43) Date of publication of application: **29.06.2005**
(21) Application number: 03756607.2
(22) Date of filing: 30.09.2003
(51) Int. Cl.: A61K 31/401, A61P 17/02, A61P 43/00

(54) **PREVENTIVE OR REMEDY FOR BEDSORE**

(30) Priority: 30.09.2002 JP 2002285875
(71) Applicant: KYOWA HAKKO KOGYO CO., LTD., Chiyoda-ku, Tokyo 100-8185 (JP)
(72) Inventor: TAKEDA, Toshiaki, Iwate-gun, Iwate 020-0173 (JP); SHIMADA, K., Kyowa Hakko Kogyo Co, Ltd., Head Off., Chiyoda-ku, Tokyo 100-8185 (JP); KAWABE, Hideo, Kyowa Hakko Kogyo Co, Ltd, Head Off, Chiyoda-ku, Tokyo 100-8185 (JP); SHIBASAKI, Takeshi, Kyowa Hakko Kogyo Co. Ltd,, Chiyoda-ku, Tokyo 100-8185 (JP); TAKAHASHI, Tomoya, Kyowa Engineering Co., Ltd., Chiyoda-ku, Tokyo 102-0074 (JP)
(74) Representative: Williams, Aylsa
(86) International application number: PCT/JP2003/012525
(87) International publication number: WO 2004/028531

(57) **Abstract**

In accordance with the present invention, there are provided a preventive or of preventing or therapeutic agent for decubitus comprising an N-acylated derivative of hydroxyproline or a salt thereof; the above preventive or therapeutic agent wherein the N-acylated derivative of hydroxyproline or a salt thereof is contained in an amount of 0.1 to 15% by weight to the total weight; and the preventive or therapeutic agent for decubitus wherein the N-acylated group of the N-acylated derivative of hydroxyproline is an N-acylated group having 1 to 24 carbon atoms.

## Description

### Technical Field

The present invention relates to a preventive or therapeutic agent for decubitus.

### Background Art

Decubitus is a state of disease in which peripheral blood vessel of the tissue is clogged by oppression from contacting area of the body resulting in necrosis of the tissue and there are many cases where a long period is needed for its cure.

Therefore, decubitus (the so-called bedsore) is apt to happen in aged people and patients under medical treatment for a long period who are on bed for long term and that is a big problem in nursing. In addition, decubitus is a disease which occurs in animals other than human being as well and, for example, saddle sore of horses is also a kind of decubitus.

As a therapeutic agent for decubitus, ointment containing bromelain (*Shinryo* to *Shinyaku,* 1971, vol. 8, p. 967), ointment containing bucladesine sodium (Biological & *Pharmaceutical Bulletin,* 1995, vol. 18, p. 1539-1543), etc. have been used but all of them have been known to have side effects such as bleeding and pain, and there has been a demand for safer therapeutic agents for decubitus.

Moreover, in the conventional therapeutic agent for decubitus, there is no effect of preventing the generation of decubitus.

An agent for external application containing N-acetyl derivative of hydroxyproline has been used in Europe as a therapeutic agent for wound. In addition, N-acetyl derivative of hydroxyproline has been known to have a promoting action for ceramide synthesis (WO 02/6255), a moisturizing action, a suppressive action for aging and an improving action for skin quality (Japanese Published Unexamined Patent Application 80321/2002) and a promoting action for collagen synthesis (WO 00/51561) but it has not been known yet to have a preventive and therapeutic action for decubitus.

### Disclosure of the Invention

An object of the present invention is to provide a highly-safe preventive or therapeutic agent for decubitus.

The present invention relates to the following (1) to (9).
(1) A preventive or therapeutic agent for decubitus, which comprises an N-acylated derivative of hydroxyproline or a salt thereof.
(2) The preventive or therapeutic agent for decubitus according to the above (1), wherein the N-acylated derivative of hydroxyproline or a salt thereof is contained in an amount of 0.1 to 15% by weight to the total weight.
(3) The preventive or therapeutic agent for decubitus according to the above (1) or (2), wherein an acyl group of the N-acylated derivative of hydroxyproline is the acyl group having 1 to 24 carbon atoms.
(4) Use of an N-acylated derivative of hydroxyproline or a salt thereof for the manufacture of a preventive or a therapeutic agent for decubitus.
(5) The use of the N-acylated derivative of hydroxyproline or a salt thereof for the manufacture of a preventive or therapeutic agent for decubitus according to the above (4), wherein the N-acylated derivative of hydroxyproline or a salt thereof is contained in an amount of 0.1 to 15% by weight to the total weight.
(6) The use of the N-acylated derivative of hydroxyproline or a salt thereof according to the above (4) or (5), wherein the acyl group of N-acylated derivative of hydroxyproline is the acyl group having 1 to 24 carbon atoms.
(7) A method of preventing or treating decubitus, which comprises administrating a composition comprising an N-acylated derivative of hydroxyproline or a salt thereof.
(8) The method of preventing or treating decubitus according to the above (7), wherein the composition contains 0.1 to 15% by weight of the N-acylated derivative of hydroxyproline or a salt thereof to the total weight.
(9) The method of preventing or treating decubitus according to the above (7) or (8), wherein the acyl group of the N-acylated derivative of hydroxyproline is the acyl group having 1 to 24 carbon atoms.

An example of the acyl group of the N-acylated derivative of hydroxyproline used in the present invention is a linear or branched acyl group having 1 to 24 carbon(s) and its specific examples are formyl group, acetyl group, propionyl group, butyl group, isobutyl group, valeryl group, pivaloyl group, hexanoyl group, heptanoyl group, octanoyl group, nonanoyl group, decanoyl group, undecanoyl group, dodecanoyl group and the like. Preferred examples are acetyl group, propionyl group, butyl group and isobutyl group and the still more preferred example is acetyl group.

With regard to N-acylated derivative of hydroxyproline used in the present invention, an N-acylated derivative of stereoisomer of hydroxyproline may be also listed. As the stereoisomer of hydroxyproline, mention may be made of cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

As the salt of the N-acylated derivative of hydroxyproline, mention may be made of alkaline metal salts such as sodium, potassium , lithium, etc., alkaline earth metal salts such as calcium, magnesium, etc., ammonium salt, amine addition salt such as monoethanolamine, diethanolamine, triethanolamine, triisopropanolamine, etc. and basic amino acid addition salt such as arginine and lysine, etc.

The N-acylated derivative of hydroxyproline is able to be prepared by a known method. For example, the N-acylated derivative of hydroxyproline is able to be prepared in such a manner that a linear or branched and saturated or unsaturated fatty acid having 1 to 24 carbon atoms is converted to a halide such as chloride, bromide, etc. using a halogenating agent such as thionyl chloride, phosgene, etc. and then condensed with hydroxyproline or that fatty acid is converted to acid anhydride and then made to react with hydroxyproline.

With regard to the fatty acid, a fatty acid such as formic acid, acetic acid, propionic acid, butyric acid, isobutyric acid, valeric acid, isovaleric acid, pivalic acid, hexanoic acid, heptanoic acid, octanoic acid, nonanoic acid, decanoic acid, undecanoic acid, dodecanoic acid, etc. is used either solely or jointly.

A process for producing N-acylated derivative of hydroxyproline via an acid halide is exemplified as follows.

Fatty acid is dispersed in a solvent such as methylene chloride, chloroform, carbon tetrachloride, benzene, toluene, xylene, n-hexane, etc. and 1 to 5 equivalents of a halogenating agent is added thereto followed by the reaction to give a fatty acid halide. Separately, hydroxyproline is dissolved or dispersed in a solvent and, during the resulting solution is kept at 5 to 70°C, the above fatty acid halide is added in an amount of 0.3 to 3. 0 equivalent (s) to hydroxyproline to conduct an acylation reaction whereupon an N-acylated derivative of hydroxyproline is produced.

As the solvent used for the acylation reaction, mentioned may be made of water, methanol, ethanol, isopropanol, isobutanol, acetone, toluene, tetrahydrofuran, ethyl acetate, N,N-dimethylformamide, dimethyl sulfoxide and the like, and each of them may be used solely or jointly. In dissolving or dispersing hydroxyproline in a solvent, 0.8 to 2.0 equivalents of alkaline substance such as sodium hydroxide, potassium hydroxide, etc. may be dissolved or dispersed in a solvent if necessary.

In order to obtain a salt of the N-acyl derivative of hydroxyproline, the product itself may be purified when an N-acylated derivative of hydroxyproline is prepared in a form of salt while, when it is prepared in a free form, it may be dissolved or suspended in an appropriate solvent and a base is added thereto to form a salt.

With regard to purification, a conventional method such as crystallization, chromatography, etc. may be used.

As the N-acylated derivative of hydroxyproline, mention may be made of N-acetyl-cis-4-hydroxy-L-proline, N-acetyl-cis-4-hydroxy-D-proline, N-acetyl-cis-3-hydroxy-L-proline, N-acetyl-cis-3-hydroxy-D-proline, N-acetyl-trans-4-hydroxy-L-proline, N-acetyl-trans-4-hydroxy-D-proline, N-acetyl-trans-3-hydroxy-L-proline, N-acetyl-trans-3-hydroxy-D-proline, N-propionyl-cis-4-hydroxy-L-proline, N-propionyl-cis-4-hydroxy-D-proline, N-propionyl-cis-3-hydroxy-L-proline, N-propionyl-cis-3-hydroxy-D-proline, N-propionyl-trans-4-hydroxy-L-proline, N-propionyl-trans-4-hydroxy-D-proline, N-propionyl-trans-3-hydroxy-L-proline, N-propionyl-trans-3-hydroxy-D-prolne, N-butyryl-cis-4-hydroxy-L-proline, N-butyryl-cis-4-hydroxy-D-proline, N-butyryl-cis-3-hydroxy-L-proline, N-butyryl-cis-3-hydroxy-D-proline, N-butyryl-trans-4-hydroxy-L-proline, N-butyryl-trans-4-hydroxy-D-proline, N-butyryl-trans-3-hydroxy-L-proline, N-butyryl-trans-3-hydroxy-D-proline, N-isobutyryl-cis-4-hydroxy-L-proline, N-isobutyryl-cis-4-hydroxy-D-proline, N-isobutyryl-cis-3-hydroxy-L-proline, N-isobutyryl-cis-3-hydroxy-D-proline, N-isobutyryl-trans-4-hydroxy-L-proline, N-isobutyryl-trans-4-hydroxy-D-proline, N-isobutyryl-trans-3-hydroxy-L-proline, N-isobutyryl-trans-3-hydroxy-D-proline, and the like.

The preventive or therapeutic agent for decubitus according to the present invention may include one or more N-acylated derivative (s) of hydroxyproline or salt (s) thereof selected from the group consisting of N-acylated derivatives of cis/trans-4-hydroxy-L/D-proline and cis/trans-3-hydroxy-L/D-proline and salts thereof.

The preventive or therapeutic agent for decubitus according to the present invention may be used as a composition of medicament, food or drink, feed for animals, food additive, feed additive and the like and, preferably, it may be used as a medicament.

When the preventive or therapeutic agent for decubitus according to the present invention is used as a medicament, it is possible that the N-acylated derivative of hydroxyproline or a salt thereof which is an active ingredient is administered solely, but it is usually preferred to administer as a pharmaceutical preparation which is manufactured by any method which has been well known in the technical field of pharmaceutical science by mixing the active ingredient with one or more pharmacologically acceptable carrier(s).

With regard to a method of administration, it is desirable to select the most effective method for prevention or treatment of decubitus. The administering method includes, for example, parenteral administration such as application, subcutaneous, intramuscular, intravenous, oral, intra-airway and intrarectal administration, etc. as well as oral administration and the like. Parenteral administration is preferred and application or subcutaneous administration is more preferred. As the dosage form, mention may be made of ointment, tape, spray, injection, suppository, capsule, tablet, granule, syrup, emulsion and the like.

As the pharmaceutical preparation suitable for parenteral administration of the preventive or therapeutic agent according to the present invention, mention may be made of agents for external application, injection and suppository and, an agent for external application is more preferred.

With regard to the form of the agent for external application, there is no particular limitation so far as it contains N-acylated derivative of hydroxyproline or a salt thereof and a base and is in a form which is applicable to the diseased area and its examples include liquid, aerosol, cream, gel (jelly), powder, ointment, poultice and liniment. Liquid, aerosol, cream, gel, ointment, poultice, etc. are preferred.

Depending upon its form, the agent for external application may be prepared using a commonly used base such as oily base or hydrophobic base, emulsion-type base, hydrophilic base or water-soluble base, gel base, etc. and a commonly used component such as surfactant, fatty acid or derivative thereof, ester of polycarboxylic acid with alcohol, higher alcohol, suspending agent or thickener, powder/granular inorganic substance, gel-forming substance, water, alcohol, polyhydric alcohol, alkanolamine, buffer, propellant and the like.

Examples of components for oily base or hydrophobic base are Vaseline, liquid paraffin, paraffin wax, plasti-base containing liquid paraffin and polyethylene, silicone oil, triglyceride, squalene, beeswax, bleached beeswax, microcrystalline wax, paraffin wax, whale wax and other wax and pure lanoline. Examples of components for emulsion-type base are oily base such as Vaseline, lanoline, etc., higher alcohol, surfactant, emulsifier and the like. As the emulsifier, mention may be made of nonionic and anionic surfactants, cholesterol, higher alcohol, free fatty acid derived from plant oil and the like.

Examples of components for hydrophilic base or water-soluble base are hydrophilic fatty acid ester such as glycerol ester of saturated fatty acid (for example, Adeps solidus and the like) and water-soluble polymer such as polyethylene glycol (for example, Macrogol, etc.) and the like.

Examples of components of gel base are organic hydrogel base such as colloid-dispersed starch, tragacanth, alginate, cellulose derivative (for example, methyl cellulose, carboxymethyl cellulose, carboxymethyl cellulose sodium, etc.), etc., and inorganic hydrogel base containing colloidal clay (for example, silicates such as bentonite, Veegum, etc.) and the like.

As the surfactant, mention may be made of natural emulsifier such as gum acacia, gelatin, tragacanth, lecithin , cholesterol, etc. anionic surfactants such as soap and sodium alkyl sulfate, nonionic surfactant such as polyoxyethylene sorbitan fatty acid ester (for example, monooleyl polyoxyethylene sorbitan, etc.), polyoxyethylene castor oil derivative, polyoxyethylene hydrogenated castor oil, glycerol fatty acid ester (for example, glycerol monostearate, sorbitan monooleate, etc.), sorbitan fatty acid ester (for example, sorbitan monostearate and sorbitan sesquistearate), polyoxyethylene higher alcohol ether (for example, polyoxyethylene cetyl ether, etc.), polyoxyethylene alkyl phenol, polyoxyethylene oxypropylene copolymer (for example, Pluronic, etc.) and the like, cationic surfactants such as cetyl trimethylammonium chloride, etc., amphoteric surfactant and the like.

As the fatty acid or derivative thereof, mention may be made of higher fatty acids such as oleic acid, stearic acid, etc., and a salt thereof; ester of higher fatty acid such as caprylic acid, caproic acid, myristic acid, palmitic acid, stearic acid, oleic acid, etc. with monohydric aliphatic alcohol (for example, isopropyl myristate, isopropyl palmitate, isopropyl stearate, decyl oleate, etc.); triglycerides such as caprylic acid triglyceride, caproic acid triglyceride, peanut oil, castor oil, cacao oil and hydrogenated fat/oil (for example, hydrogenated castor oil, etc.) and the like; and fatty acid ester of polyhydric alcohol such as pentaerythritol fatty acid ester, etc.

As the ester of polycarboxylic acid with alcohol, mention may be made of ester of polycarboxylic acid such as adipic acid and sebacic acid with monohydric aliphatic alcohol (for example, ethyl adipate, diisopropyl adipate, etc.) and the like.

As the higher alcohol, mention may be made of benzyl alcohol, lauryl alcohol, myristyl alcohol, cetyl alcohol, stearyl alcohol, cetostearyl alcohol, 2-octyldodecanol, etc.

As the suspending agent and thickener, mentioned may be made of polysaccharides such as gum acacia, tragacanth, pullulan, locust bean gum, bean gum, pectin, xanthan gum, guar gum, etc., methyl cellulose, carboxymethyl cellulose, polyvinyl alcohol, polyvinylpyrrolidone, acrylic acid copolymer, carboxyvinyl polymer, colloidal microcrystalline cellulose and the like.

As the powdery/granular inorganic substance, mention may be made of talc, silicic acid anhydride, calcium carbonate, magnesium carbonate, colloidal silica, bentonite, etc.

As the gel-forming substance, mention may be made of polyacrylic acid, polymethacrylic acid or salt thereof, highly water-absorbing resin such as cross-linked polyvinyl alcohol, carboxymethyl cellulose sodium, gum acacia, xanthan gum and guar gum.

The alcohol includes, for example, ethanol, isopropanol and the like.

The polyhydric alcohol includes, for example, ethylene glycol, diethylene glycol, propylene glycol, dipropylene glycol, tripropylene glycol, 1,3-tetramethylene glycol, glycerol, sorbitol and the like.

The alkanolamine includes, for example, diethanolamine, triethanolamine and the like.

The buffer includes, for example, citrate, malate, acetate, phosphate, polar amino acid, sodium hydroxide and the like.

As the component for propellant, mention may be made of low-boiling fluorinated hydrocarbon (such as CFC 22), aliphatic hydrocarbon (such as propane, butane, etc.) and the like.

For external application, if necessary, other additive such as preservatives, e.g. p-hydroxybenzoate ester(for example, methyl p-hydroxybenzoate, propyl p-hydroxybenzoate, etc.), stabilizer, e.g., antioxidants (for example, butylhydroxytoluene, etc.), coloring agents and fragrance-giving agents may be added to the agent.

When the agent for external application is liquid, it is also possible to use surfactants and emulsifiers and, if necessary, higher fatty acid ester, higher alcohol, suspending agenst or thickeners, alcohols, polyhydric alcohols, preservative, etc. In an aerosol agent, a propellant is used together with the above components for liquid preparation and, if necessary, it is also possible to use a solvent such as ethanol, glycerol, propylene glycol, etc., higher fatty acid ester, surfactants and the like.

The gel preparation contains a gel-forming agent and, with regard to a base for cream and ointment, the above-mentioned oily base or hydrophobic base, emulsion-type base, hydrophilic base or water-soluble base and gel base are used.

The powdery preparation may be prepared by using excipients such as lactose, starch, etc., binder disintegrating agents and other appropriate additives.

With regard to the component of base for poultice, Japanese Published Unexamined Patent Application 105630/1993 may, for example, be referred to and its examples are rubber components such as styrene-isoprene-styrene block copolymer and styrene-isoprene-styrene block copolymer, etc., tackiness-giving agents, oil components, water-soluble polymer and water-absorbing polymer, water and antioxidants.

In the liniment, it is possible to use oil emulsion such as fat oil, soap, gum acacia, tragacanth, etc., and alcohol soap and the like, and if necessary, glycerol, carmellose sodium, etc. may be used as well.

The above-mentioned base and the above-mentioned component in those preparations may be appropriately selected depending upon the type of the preparation and the amount thereof may also be appropriately selected within a range which is usually used in the preparation.

The agent for external application may be applied by, for example, means of application, inunction or spraying depending upon dosage form, etc. Applying amount of the agent for external application to the diseases area may be selected by, for example, amount of the active ingredient. Thus, for example, 0.02 to 100 mg, preferably 0.2 to 20 mg or, particularly preferably, 2 to 10 mg is applied per cm² of the diseased area per day once to about three times daily. Although there is no particular limitation for the period of administration, it is usually from one day to one year and, preferably, from one week to three months.

The amount of N-acylated derivative of hydroxyproline or a salt thereof contained in the above preparation is 1 to 1,000 mg, preferably 1 to 500 mg or, more preferably, 1 to 200 mg in 1 g of the above preparation.

Injection preparation is prepared using a carrier, etc. comprising a salt solution, a glucose solution or a mixture thereof. Suppository is prepared using a carrier such as cacao butter, hydrogenated fat, carboxylic acid, etc.

Examples of preparations suitable for oral administration are tablet, diluted powder, granule, emulsion, syrup, capsule, etc. When the dosage form of the oral preparation is tablet, diluted powder, granule, etc., it is possible to prepared the preparation by addition of excipients such as saccharide (for example, lactose, sugar, glucose, sucrose, mannitol, sorbitol, etc.), starch (for example, that of potato, wheat, corn, etc.), inorganic substance (for example, calcium carbonate, calcium sulfate, sodium hydrogen carbonate, sodium chloride, etc.) and plant powder (for example, licorice powder, gentian powder, etc.), disintegrating agents such as starch, agar, gelatin powder, crystalline cellulose, carmellose sodium, carmellose calcium, calcium carbonate, sodium hydrogen carbonate, sodium alginate, etc., lubricants such as magnesium stearate, talc, hydrogenated plant oil, Macrogol, silicone oil, etc., binders such as polyvinyl alcohol, hydroxypropyl cellulose, methyl cellulose, ethyl cellulose, carmellose, gelatin, starch paste solution, etc., surfactants such as fatty acid ester, plasticizer such as glycerol, etc. and the like.

When the dosage form of the oral preparation is liquid preparation such as syrup, etc., it is able to prepare the preparation by adding water, saccharides such as sucrose, sorbitol, fructose, etc., glycols such as polyethylene glycol, propylene glycol, etc., oils such as sesame oil, olive oil, soybean oil, etc., antiseptics such as p-hydroxybenzoate ester, etc., flavors such as straw berry flavor, peppermint, etc. and the like.

Although dose of the above-mentioned injection and oral preparation to human being varies depending upon dosage form, degree of symptom, age and body weight of a patient, etc., it is usually 0.02 to 100 mg, preferably 0.2 to 20 mg or, particularly preferably, 2 to 10 mg per kg body weight of human being per day. Although there is no particular limitation for the period for administration, it is usually from one day to one year and, preferably, from one week to three months.

Amount of the N-acylated derivative of hydroxyproline or a salt thereof contained in the above preparation is 1 to 1,000 mg, preferably 1 to 500 mg or, more preferably, 1 to 150 mg per g of the preparation.

Incidentally, in the present invention, "prevention of decubitus" means that, by way of usual administration or ingestion of the preventive agent for decubitus of the present invention, the effect such as (a) complete prevention of decubitus, (b) reduction in incidence rate or (c) suppression of symptom upon onset is achieved.

The preventive agent or the treating agent for decubitus according to the present invention is able to be used not only for human being but also for animals except human being. Examples of the animals except human being are dog, cat, cattle, horse, etc.

Although the dose of the preventive or therapeutic agent for decubitus according to the present invention varies depending upon dosage form, type of the animal to which the agent is administered, age and body weight of the animal, etc. , it is usually 0.02 to 100 mg, preferably 0.2 to 20 mg or, particularly preferably, 2 to 10 mg per kg body weight of the animal per day. Although there is no particular limitation for the administering period, it is usually one day to one year or, preferably, from one week to three months.

When the preventive or therapeutic agent for decubitus according to the present invention is used as food or drink, a product where the N-acylated derivative of hydroxyproline or a salt thereof is added to food or drink is able to be used as food or drink for prevention or treatment of decubitus.

The food or drink as such is able to be processed/prepared by a common method for the manufacture of food or drink except addition of the N-acylated derivative of hydroxyproline or a salt thereof.

When the preventive or therapeutic agent for decubitus is used for food or drink, its form may be any of juice, refreshing drink, tea, lactic acid bacteria beverage, milk product such as fermented milk, ice cream, butter, cheese, yogurt, processed milk, skim milk, etc., meat product such as ham, sausage, hamburger, etc., fish meat paste product such as *kamaboko, chikuwa, Satsuma-age*, etc., egg product such as *dashi-maki*, steamed egg custard, etc., confectionery such as cookie, jelly, chewing gum, candy, snack, etc., bread, noodle, pickles, smoked product, dried product, *tsukudani*, salted product, soup, seasoning, etc.

Form of the food or drink may also be any of powdery food, sheet-shaped food, bottled food, canned food, retort food, capsule food, food or drink in a tablet form, fluid food, drink, etc.

The preventive or therapeutic agent for decubitus according to the present invention may also be used as health food or functional food having an effect of preventing or treating decubitus.

When the food or drink is a beverage or a tablet, it is prepared by such a manner that other components, additive, etc. are added, if necessary, to the N-acylated derivative of hydroxyproline or a salt thereof followed by dissolving or dispersing in an appropriate amount of water or making into tablets. In the case of confectionery such as candy, drop, chocolate, jelly, biscuit, cookie, etc., other components, additive, etc. are added to the N-acylated derivative of hydroxyproline or a salt thereof and, further necessary, an appropriate carrier such as wheat flour, rice flour, starch, corn starch, soybean, etc. is added thereto followed by making into an appropriate shape by a conventional method to give a product.

The above food or drink may also be prepared by means of a granulating method such as fluidized bed granulation, stirring granulation, extrusion granulation, tumbling granulation, air current granulation, compression molding granulation, disintegration granulation, spray granulation, jet granulation, etc., a coating method such as pan coating, fluidized bed coating, dry coating, etc., a swelling method such as puff dry, excessive steam method, foam mat method, microwave heating method, etc., an extrusion method such as extrusion granulation, extruder, etc. and the like.

When the preventive or therapeutic agent for decubitus according to the present invention is used as a food additive, such a food additive is able to be prepared by the same method as in the case of the above-mentioned oral administration. Usually, the food additive is mixed with or dissolved in other food additive and processed/produced into a form of, for example, powder, granule, pellet, tablet or various liquid preparations.

Food additive which is commonly used for food or drink such as sweetener, coloring agent, preservative, thickener/stabilizer, antioxidant, color coupler, bleaching agent, antifungal agent, gum base, bitter agent, enzyme, brightener, acidifying agent, seasoning, emulsifier, enriching agent, agent for manufacture, flavoring ingredient, spice extract, etc. may be added to the above-mentioned food or food additive.

Adding amount of the N-acylated derivative of hydroxyproline or a salt thereof or a food additive to the above-mentioned food or drink is appropriately selected depending upon type of food or drink, effect expected by ingestion of the food or drink, etc. and, as the N-acylated derivative of hydroxyproline or a salt thereof, it is usually added so as to make its content 0.1 to 100% by weight, preferably 0.1 to 50% by weight or, more preferably, 0.1 to 15% by weight.

Ingesting amount of the above-mentioned food or drink varies depending upon ingesting form, age and body weight of the person who ingests it, etc., it is usually 0.02 to 100 mg, preferably 0.2 to 20 mg or, particularly preferably, 2 to 10 mg as the N-acylated derivative of hydroxyproline or a salt thereof per day for an adult and it is ingested once or several times a day. Although there is no particular limitation for the ingesting period, it is usually from one day to one year or, preferably, from one week to three months.

When the food or drink is routinely ingested, decubitus is able to be prevented.

When one is attacked with decubitus already, the food or drink is routinely ingested whereby decubitus is able to be treated such as that symptom of decubitus is alleviated or cured.

When the preventive or therapeutic agent for decubitus according to the present invention is used as a feed for animals, a product prepared by addition of the N-acylated derivative of hydroxyproline or a salt thereof to the feed for animals may be used as a feed for animals for prevention or treatment of decubitus. Such a feed for animals is able to be processed/produced by a common manufacturing method for feed.

With regard to the feed for animals, any feed may be used as far as it is a feed having a preventive effect for decubitus of animals except human being and its examples are feed for pets such as dogs, cats, etc., pet food, feed for cattle, horse, etc. and the like.

Such a feed for animals is able to be used as a supplementary food for health of animals having an effect of preventing or treating of decubitus.

Examples of the feed are cereals, chaff and bran, plant oil cake, feed derived from animals and other feed, purified product, a mixture thereof, etc.

Examples of cereals are milo, wheat, barley, oat, rye, unpolished rice, buckwheat, foxtail millet, broomcorn millet, Japanese millet, corn, soybean, etc.

Examples of chaff and bran are rice bran, defatted rice bran, wheat bran, rice flour, wheat, embryo, barley bran, pellet, corn bran, corn embryo, etc.

Examples of plant oil cake are soybean oil cake, soybean flour, linseed oil cake, cottonseed oil cake, peanut oil cake, safflower oil cake, coconut oil cake, palm oil cake, sesame oil cake, sunflower oil cake, rapeseed oil cake, kapok oil cake, mustard oil cake, etc.

Examples of feed derived from animals are fish powder such as Northern ocean meal, imported meal, whole meal, etc., coast meal, fish soluble, meat powder, meat bone powder, blood powder, degraded hair, bone powder, side product upon treatment of animals, feather meal, chrysalis of silk worm, skim milk, casein, dry whey, etc.

Examples of other feed are plant stems/leaves such as alfalfa, hay cube, alfalfa leaf meal, black locust powder, etc, side products in corn process industry such as corn gluten, meal, corn gluten feed, corn steep liquor, etc., processed starch such as starch, etc. , fermentation industry product such as yeast, beer cake, malt root, alcohol cake, soy sauce cake, etc., side product in agricultural manufacture such as cake after processing of citrus fruits, soybean curd cake, coffee grounds; cocoa grounds, etc., cassava, broad bean, guar meal, sea algae, krill, spirulina, chlorella, minerals, etc.

Examples of pure product are protein such as casein, albumin, etc., amino acid, starch, cellulose, saccharide such as sucrose, glucose, etc., minerals, vitamins and the like.

The above-mentioned feed for animals is also able to be produced by a granulation method such as fluidized bed granulation, stirring granulation, extrusion granulation, tumbling granulation, air current granulation, compression molding granulation, disintegrating granulation, spray granulation, jet granulation, etc., coating method such as pan coating, fluidized bed coating, dry coating, etc., swelling method such as puff dry, excessive steam method, foam mat method, microwave heating method, etc., extrusion method such as extrusion granulator, extruder, etc. and the like.

Feed additive may be prepared by the same method as that for the oral preparation. Feed additive is usually mixed with or dissolved in other feed additive if necessary and is processed/produced in the form of, for example, powder, granule, pellet, tablet and various liquid preparations.

Amount of N-acylated derivative of hydroxyproline or a salt thereof contained in the feed for animals varies depending upon ingesting mode, type of the animal ingesting it, age, body weight, etc. of the animal and, usually, it is added so as to make the daily ingesting amount 0.02 to 100 mg, preferably 0.2 to 20 mg or, particularly preferably, 2 to 10 mg per kg body weight of the animal to be administered.

The feed for animals is ingested once or several times a day. It is also possible that the feed additive of the present invention is administered as an oral preventive or therapeutic agent for animals for decubitus where the ingesting animal is a subject in the same dose and administering period as the ingesting amount and ingesting period for the above-mentioned feed.

When the feed for animals is routinely administered to animals, decubitus is able to be prevented.

When decubitus is already noted, the feed for animals is routinely administered whereby decubitus is able to be treated.

### Brief Description of Drawings

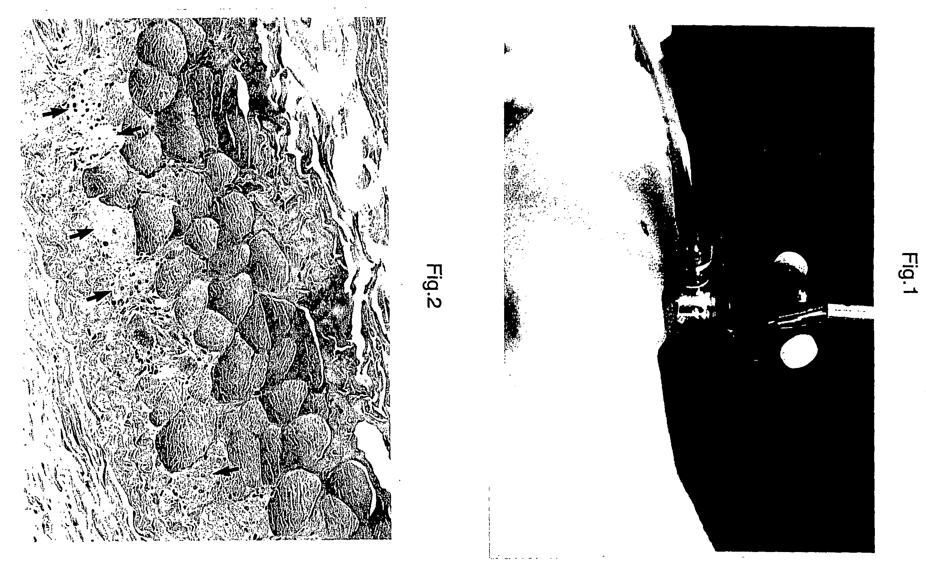

Fig. 1 is a drawing which shows a balloon pressure apparatus.
Fig. 2 is a drawing which shows a tissue image of the site to which a base is applied. At the site to which an arrow shows, denatured necrosis image of cutaneous muscle is noted.
Fig. 3 is a drawing which shows a tissue image of the site to which an N-acetylhydroxyproline preparation was applied.
Fig. 4 is a drawing which shows an increase/decrease ratio of water content in horny layer of the skin in the site applied with a base cream or an AHYP cream in the waist of rabbit of malnutrition. In the drawing, black columns and open columns are test groups to which a base cream and an AHYP cream were applied, respectively. An ordinate shows an increasing rate (%) to the water content in the horny layer of the skin before application of the cream while an abscissa shows elapsed days from initiation of application of the cream.

### Best Mode for Carrying Out the Invention

The examples of the invention are described below.

### Example 1. Preventive effect of the N-acylated derivative of hydroxyproline to decubitus (1)

A glycerol preparation containing 10% by weight of the N-acetylhydroxyproline (hereinafter, referred to as the AHYP preparation) and a glycerol preparation containing no N-acetylhydroxyproline (control preparation; hereinafter, referred to as the base) were prepared by a conventional method. Compositions of the AHYP preparation and of the base are shown in Table 1. In Table 1, the term "q.s." means an amount of sodium hydroxide added necessary for making the pH of the AHYP preparation or glycerol preparation 5.99 and the term "balance" means an amount of purified water added necessary for making 100% by weight as a preparation by addition of purified water to the total amount of components other than purified water.

**Table 1**

| Names of Components | AHYP Preparation (% by weight) | Base (% by weight) |
|---|---|---|
| N-Acetylhydroxyproline | 10 | 0 |
| Glycerol | 30 | 30 |
| Citric acid | 0.3 | 0.3 |
| Sodium hydroxide | q.s. | q.s. |
| Purified water | balance | balance |
| Total | 100 | 100 |
| pH | 5.99 | 5.99 |
| Property | transparent | transparent |

In the animal experiment, 3 healthy male rabbits of Japanese white species (purchased from Kitayama Rabesu) were used. Body weight of the animals used for the experiment was 3,200 to 3,500 g.

Dorsolumbar skin of the rabbits was clipped by an electric hair clipper (DC5, manufactured by Shimizu Denki Kogyo) and depilated using a depilating cream [Dibale (registered trade mark), manufactured by Shiseido Cosmenity]. After that, 0.2 ml of the AHYP preparation was applied to the skin on right iliac wing of about 30 cm² twice daily for continued four days. A base was similarly applied to the skin on left iliac wing of the same animal. During the above period, redness, etc. of the skin to which application was conducted were observed by naked eye. On the next day after completion of the application, oppression was applied for 24 hours to the skin to which the test substance was applied so as to result in light decubitus. The tested sites were observed by naked eye and photographic pictures were taken immediately after removal of oppression and after 24 hours therefrom. After 24 hours from removal of oppression, the oppressed skin was excised and fixed with a 20% neutral buffer formalin solution (manufactured by Wako Pure Chemical).

The excised oppressed skin was fixed for 5 days with the formalin solution and then cut out in stripes to prepare pathological specimens. Incidentally, the tissue specimens were prepared at the Histological Science Laboratory (984-1, Kurosawa-4-chome, Ohme-shi, Tokyo).

When the oppressed skin was observed, no skin irritation such as redness was noted on the skin by application of the test substance.

Immediately after removal of oppression by a balloon, a light redness was noted in the case of application of any of the AHYP preparation and the base. After 24 hours from removal of oppression, the redness disappeared.

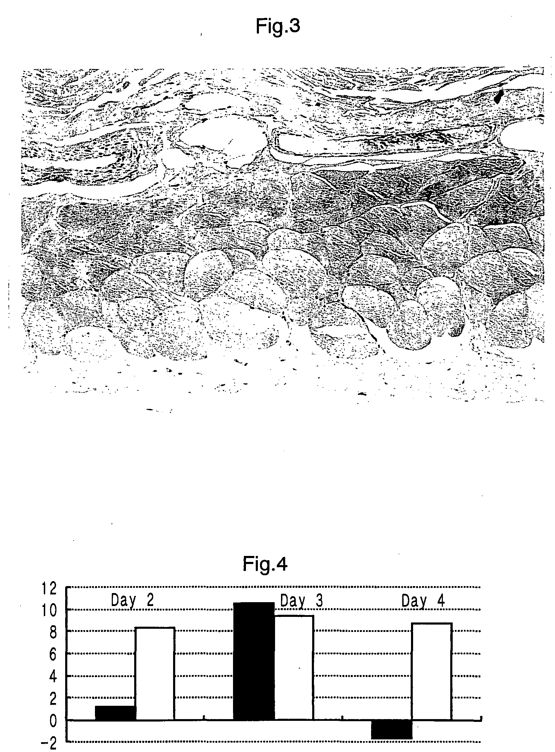

When the site to which a base was applied was subjected to a histological test, denaturation/necrosis image of cutaneous skin was observed together with limited wound image of epidermis (Fig. 2). On the other hand, in the histological test of the site to which the AHYP preparation was applied, although the wound image of epidermis was noted as well, denaturation/necrosis image of cutaneous skin was not noted (Fig. 3). Thus, it was confirmed that, in the lesion to which the AHYP preparation was applied, degree of wound was weak as compared with the control lesion to which the base was applied.

From the above result, it was found that the N-acylated derivative of hydroxyproline had a preventive effect for decubitus.

### Example 2. Preventive effect of the N-acylated derivative of hydroxyproline to decubitus (2)

A preventive effect of the N-acylated derivative of hydroxyproline for decubitus was tested using rabbits in which malnutrition was induced (hereinafter, referred to as rabbits of malnutrition) which is one of the causes for resulting weak skin.

The rabbits of malnutrition were prepared by breeding of three male healthy rabbits of Japanese white species having body weight of 3,200 to 3,500 g for 15 days to which tap water was freely given but no feed was given. Body weight of rabbits of malnutrition decreased to an extent of about 20% as compared with before fasting. In a hematobiochemical test, both total protein in serum and albumin in serum decreased from 6.4 g/dl in average to 5.2 g/dl in average and from 4.4 g/dl in average to 3.2 g/dl in average, respectively as compared with before fasting.

A cream containing 10% by weight of the N-acetylhydroxyproline (hereinafter, referred to as the AHYP cream) and a cream containing no N-acetylhydroxyproline (hereinafter, referred to as the base cream) were prepared by a conventional method. Compositions of the AHYP cream and the base cream are shown in Table 2. Incidentally, in Table 2, the term "q.s." means the adding amount of purified water necessary for making the total amount of the preparation 100% by weight.

**Table 2**

| Names of Components | Base Cream (% by weight) | AHYP Cream (% by weight) |
|---|---|---|
| N-Acetylhydroxyproline | 0 | 10 |
| Emulsifier | 1.0 | 1.0 |
| Polyhydric alcohol fatty acid ester | 1.9 | 1.9 |
| Oily base | 18.0 | 18.0 |
| Polyhydric alcohol | 13.5 | 13.5 |
| Thickener | 0.1 | 0.1 |
| Antiseptic (Paraben) | 0.2 | 0.2 |
| Buffer | 1.8 | 3.7 |
| Purified water | q.s. | q.s. |
| Total Amount | 100 | |

Dorsolumbar skin of the rabbits of malnutrition were depilated, one circular site of 4 cm diameter was secured on iliac wing at the left waist and 40 mg of the AHYP cream was applied thereto twice daily nearly at the same time for continued four days. At the right waist, a base cream was applied in the same proceeding as in the case of the left waist. In order to check the moisturizing effect, water content of the horny layer of the skin to which the above was applied,was measured once daily using a moisture checker (Skin Moisture Meter MY-707S; manufactured by K. K. Scala) and averaged for each cream and degree of increase/decrease to the state before application and change in days were recorded. On the fifth day from the start of the experiment, oppression was added to the skin to which cream was applied to prepare a light decubitus using a balloon pressure apparatus by the same method as mentioned in Example 1. After that, the oppressed skin was excised and a histological test was conducted.

### (1) Moisturizing effect

Changes in water content at horny layer of the skin at the waist of the rabbits are shown in Fig. 4. At the site where the base cream was applied, a significant increasing rate was noted after 3 days from application of the cream while, on the fourth day, a decrease was noted. On the contrary, at the site where the AHYP cream was applied, an increase of around 9% was noted from the second day after application of the cream and that was maintained until the fourth day.

### (2) Histological test

The site where the AHYP cream was applied, degree of wound of epidermis of the oppressed site was light as compared with the site where the base cream was applied.

From the above results, it was noted that the N-acylated derivative of hydroxyproline has a preventive effect for decubitus in rabbits of malnutrition which is a pathological model of decubitus.

### Example 3. Therapeutic effect of the N-acylated derivative of hydroxyproline for decubitus

A therapeutic effect of the N-acylated derivative of hydroxyproline for decubitus was measured using saddle sore of horses as an subject.

An ointment containing 2.5% by weight of the N-acetylhydroxyproline was prepared by the following method. Incidentally, in Table 3, the term "balance" means an adding amount of purified water necessary for making 100% as the preparation when purified water was added to the total amount of components other than pure water.

**Table 3**

| | Names of Components | Compounding Content by weight (%) |
|---|---|---|
| A | Stearic acid | 5.0 |
| | Cetanol | 2.0 |
| | Cetyl palmitate | 2.0 |
| | Trimethylolpropane trioctanoate | 10.0 |
| | Adsorbed pure lanolin | 3.0 |
| | Liquid paraffin | 5.0 |
| | Self-emulsified glycerol monostearate | 0.5 |
| | Polyoxysorbitan monostearate | 3.0 |
| B | Methyl paraben | 0.15 |
| | 1,3-Butylene glycol | 7.0 |
| | N-Acetylhydroxyproline | 2.5 |
| | Triethanolamine | 2.7 |
| | Purified water | balance |
| | Total | 100 |

The compounds listed in the column A of the above Table 3 were mixed and heated to dissolve at 80°C (hereinafter, referred to as layer A). Separately, the compounds listed in the column B of the above Table 3 were mixed and heated to dissolve at 80°C (hereinafter, referred to as layer B). The layer B was gradually poured into the layer A which was being stirred and a stirring at a medium speed was continued during 5 minutes after completion of pouring of the layer B. After that, the reaction solution was cooled with a low-speed stirring and the stirring was stopped when the temperature of the reaction solution reached 35°C to finish the reaction.

An ointment in which 2.5% by weight of the N-acetylhydroxyproline was compounded prepared hereinabove was applied for three weeks to the site of saddle score of Thoroughbred horse of seven years age where necrosis was noted at the lesion due to saddle score.

As a result, diseased area of the saddle score decreased and necrosis was treated even to an extent that proud flesh was generated and lanugo was formed. Incidentally, in the control test section to which an ointment comprising the components listed in Table 3 where no acetylhydroxyproline was contained, treating of saddle score was not noted.

From the above, it was confirmed that the N-acetylated derivative of hydroxyproline is effective for the treatment of oppressive necrosis.

### Example 4. Tablets containing the N-acetylated derivative of hydroxyproline

Tablets (200 mg per tablet) were prepared by a conventional method according to the formulation mentioned in Table 4.

**Table 4**

| Names of Components | Amount (mg) |
|---|---|
| N-Acetylhydroxyproline | 50 |
| Lactose | 90 |
| Corn starch | 30 |
| Synthetic aluminum silicate | 12 |
| Carboxymethyl cellulose calcium | 15 |
| Magnesium stearate | 3 |

### Example 5. Diluted powder containing the N-acetylated derivative of hydroxyproline

Diluted powder (550 mg per pack) was prepared according to the formulation mentioned in Table 5.

**Table 5**

| Names of Components | Amount (mg) |
|---|---|
| N-Acetylhydroxyproline | 50 |
| Lactose | 300 |
| Corn starch | 200 |

### Example 6. Hard capsule preparation containing the N-acetylated derivative of hydroxyproline

Hard capsule preparation (160 mg per capsule) was prepared according to the formulation mentioned in Table 6.

**Table 6**

| Names of Components | Amount (mg) |
|---|---|
| N-acetylhydroxyproline | 50 |
| Lactose | 60 |
| Corn starch | 30 |
| Hydroxypropyl cellulose | 20 |

Lactose (60 mg) and 30 mg of corn starch were added to and mixed with 50 mg of hydroxyproline and an aqueous solution of 20 mg of hydroxypropyl cellulose followed by kneading. After that, granules were prepared by a conventional method using an extrusion granulator. The granules were filled in gelatin hard capsule to prepare a hard capsule preparation.

Example 7. Soft capsule preparation containing the N-acetylated derivative of hydroxyproline

Soft capsule preparation (170 mg per capsule) was prepared according to the formulation mentioned in Table 7.

**Table 7**

| Names of Components | Amount (mg) |
|---|---|
| N-Acetylhydroxyproline | 50 |
| Soybean oil | 120 |

Hydroxyproline (50 mg) was added to and mixed with 120 mg of soybean oil. After that, the mixture was filled in soft capsules by a conventional method using a rotary soybean automated shaping machine to prepare soft capsules.

### Example 8. Refreshing drink containing the N-acetylated derivative of hydroxyproline

Refreshing drink (for 10 bottles) was prepared according to the compounding mentioned in Table 8.

**Table 8**

| Names of Components | Amount (g) |
|---|---|
| N-Acetylhydroxyproline | 5 |
| Vitamin C | 1 |
| Vitamin B₁ | 5 |
| Vitamin B₂ | 10 |
| Vitamin B₆ | 25 |
| Liquid sugar | 150 |
| Citric acid | 3 |
| Flavoring ingradient | 1 |

Water was added to make 1,000 ml.

### Example 9. Cookie containing the N-acetylated derivative of hydroxyproline

Cookies (for 30 cookies) were prepared according to the compounding mentioned in Table 9.

**Table 9**

| Names of Components | Amount |
|---|---|
| N-Acetylhydroxyproline | 1.5 g |
| Soft wheat flour | 100 g |
| Starch | 74 g |
| Water | 14 g |
| Baking powder | 2 small tablespoonful |
| Salt | 1/2 small tablespoonful |
| Egg | one |
| Butter | 80 g |
| Milk | 2 large tablespoonful |
| Honey | a little |

### Industrial Applicability

In accordance with the present invention, there is provided a preventive or therapeutic agent for decubitus comprising an N-acetylated derivative of hydroxyproline or a salt thereof.

## Claims

1. A preventive or therapeutic agent for decubitus, which comprises an N-acylated derivative of hydroxyproline or a salt thereof.

2. The preventive or therapeutic agent for decubitus according to claim 1, wherein the N-acylated derivative of hydroxyproline or a salt thereof is contained in an amount of 0.1 to 15% by weight to the total weight.

3. The preventive or therapeutic agent for decubitus according to claim 1 or 2, wherein an acyl group of the N-acylated derivative of hydroxyproline is the acyl group having 1 to 24 carbon atoms.

4. Use of an N-acylated derivative of hydroxyproline or a salt thereof for the manufacture of a preventive or therapeutic agent for decubitus.

5. The use of the N-acylated derivative of hydroxyproline or a salt thereof for the manufacture of a preventive or therapeutic agent for decubitus according to claim 4, wherein the N-acylated derivative of hydroxyproline or a salt thereof is contained in an amount of 0.1 to 15% by weight to the total weight.

6. The use of the N-acylated derivative of hydroxyproline or a salt thereof according to claim 4 or 5, wherein the acyl group of N-acylated derivative of hydroxyproline is the acyl group having 1 to 24 carbon atoms.

7. A method of preventing or treating decubitus, which comprises administering a composition comprising an N-acylated derivative of hydroxyproline or a salt thereof.

8. The method of preventing or treating decubitus according to claim 7, wherein the composition contains 0.1 to 15% by weight of the N-acylated derivative of hydroxyproline or a salt thereof to the total weight.

9. The method of preventing or treating decubitus according to claim 7 or 8, wherein the acyl group of N-acylated derivative of hydroxyproline is the acyl group having 1 to 24 carbon atoms.
